# EUROPÄISCHE PATENTSCHRIFT

(11) **EP 1 415 599 B1**
(45) Veröffentlichungstag und Bekanntmachung des Hinweises auf die Patenterteilung: **10.01.2007**
(21) Anmeldenummer: 03022020.6
(22) Anmeldetag: 01.10.2003
(51) Int. Cl.: A61B 17/17

(54) **Zielgerät für einen Verriegelungsnagel**
Aiming device for intramedullary nail
Appareil de visée pour clou intramédullaire

(30) Priorität: 02.11.2002 DE 20216857 U
(43) Veröffentlichungstag der Anmeldung: 06.05.2004
(73) Patentinhaber: Stryker Trauma GmbH, 24232 Schönkirchen (DE)
(72) Erfinder: Zander, Nils, 24340 Eckernförde (DE); Cremer, Axel, 23795 Fahrenkrog (DE)
(74) Vertreter: Kopf, Korbinian Paul

(56) Entgegenhaltungen:
- DE-U- 20 204 655
- US-A- 5 122 146
- US-A- 5 695 365

## Beschreibung

Die Erfindung bezieht sich auf ein Zielgerät für einen Verriegelungsnagel nach dem Oberbegriff des Anspruchs 1.

Das erfindungsgemäße Zielgerät ist besonders für Suprakondylamägel geeignet. Suprakondylamägel werden bekanntlich über das Ende der distalen Femur in den Knochenkanal eingetrieben. Sie dienen zur Versorgung von Brüchen im Kondylenbereich des Femurs. Ein derartiger Nagel ist als Verriegelungsnagel ausgeführt, d.h. er ist mit Querbohrungen versehen, durch welche Verriegelungsschrauben hindurchgeführt werden, um den Nagel sicher in der Ferner zu verankern. Zum Auffinden der Querbohrungen eines Verriegelungsnagels ist ein Zielgerät erforderlich.

Eine Kategorie der Zielgeräte bei Verriegelungsnägeln ist die, dass das Zielgerät auf ein Ende des Verriegelungsnagels aufgesetzt wird. Hierbei dient das Zielgerät zugleich als Einschlaginstrument zum Eintreiben des Nagels in den Femurkanal. Das Zielgerät hat an seinem Zielarm mindestens eine Zielbohrung, die mit einer Bohrung im Nagelschaft ausgerichtet ist. Nach dem Einschlagen des Nagels wird über die Zielbohrung der Knochen angebohrt, bevor die Verriegelungsschrauben eingeschraubt werden.

Bei Verriegelungsnägeln ist bekannt, die Querbohrungen in unterschiedlichen Winkeln und Abständen anzuordnen. Es ist ferner bekannt, die Achsen der Querbohrungen in Umfangsrichtung des Nagels um vorgegebene Winkel zu versetzen. Es ist außerdem bekannt, unterschiedlich lange Nägel vorzusehen, wobei deren Querbohrungen einen unterschiedlichen Abstand zum distalen Ende aufweisen. Wenn nicht besondere Vorkehrungen getroffen werden, wäre für jeden der unterschiedlichen Nägel ein separates Zielgerät erforderlich. Bei der Versorgung mit derartigen Nägeln würde dies zu einem unvertretbar hohen Aufwand führen.

Aus US-A-5,122,156 ist eine Führungsvorrichtung für einen Zieldraht bekannt geworden. Zieldrähte werden dazu verwendet, Implantate in einen Röhrenknochen einzufädeln. Die bekannte Vorrichtung weist eine Handhabe auf, in der eine längere Hülse axial verschieblich geführt ist. Die Hülse kann innerhalb der Handhabe durch eine Feststellschraube oder dergleichen axial festgelegt werden. Auf der Hülse ist eine weitere Hülse verschiebbar gelagert, die auf der ersten Hülse ebenfalls durch eine Feststellschraube feststellbar ist. Im freien Endbereich weist die erste Hülse eine Skalierung auf, und die zweite Hülse weist ein Fenster auf, über das die Skalierung erkannt werden kann, um die Lage der zweiten Hülse auf der ersten einzustellen.

In DE-A-202 04 655 ist ein Zielgerät beschrieben. Das Zielgerät weist eine Haltevorrichtung auf, welche einen Verriegelungsnagel mit dem Zielgerät verbindet, eine Aufnahmebohrung auf, die ihrerseits einen Haltestab aufnimmt. In der Aufnahmebohrung angeordnet erstreckt sich der Haltestab annähernd parallel zum Zielarm, in dem sich mindestens eine Zielbohrung befindet. Der Haltestab weist in dem Bereich, in dem er sich innerhalb der Zielbohrung befindet, Ansenkungen auf. Mit den Ansenkungen kann ein Verriegelungsstift in Eingriff gelangen, der radial zur Achse der Aufnahmebohrung bewegbar ist. Der Verriegelungsstift legt dadurch sowohl die axiale Lage als auch die Drehlage des Haltestabes fest. Der Haltestab weist Mittel auf zur Anbringung des zugeordneten Endes des Verriegelungsnagels. Hierzu gehört üblicherweise ein Gewindestift, der in das Ende des Nagels eingeschraubt wird, sich durch den hohlen Haltestab erstreckt und der am anderen Ende mit Hilfe einer Mutter unter Zugspannung gesetzt wird, damit Nagel und Haltestift fest gegeneinander gezogen werden. Miteinander zusammenwirkende Mittel von Nagel und Haltestift legen außerdem eine vorgegebene Drehlage dieser Teile zueinander fest.

Der Verriegelungsstift wird von einer Handhabe betätigt, um wahlweise einen Eingriff mit einer Ansenkung herbeizuführen oder den Verriegelungsstift zu entriegeln. Die Lage der Ansenkungen im Haltestift ist derart, dass die Zielbohrung im Zielarm zu einer Querbohrung des Nagels ausgerichtet ist, wenn sich der Verriegelungsstift in einer Ansenkung befindet.

Der Hersteller von Verriegelungsnägeln unterschiedlicher Abmessungen bzw. unterschiedlicher Anordnung der Querbohrungen wird naturgemäß nur einen Satz derartiger Nägel herstellen. Mit diesem Satz sollen alle normalerweise vorkommenden Versorgungsfälle abgedeckt werden. Demzufolge braucht der Haltestab auch nur maximal die Anzahl von Ansenkungen aufweisen, die zu den Querbohrungen der einzelnen Nägel eines Satzes passen. Natürlich ist auch denkbar, mehrere Haltestäbe vorzusehen, die zu bestimmten Verriegelungsnägeln passen.

Der Handhabe sind erfindungsgemäß Mittel zugeordnet, die anzeigen, ob das Verriegelungselement bzw. der -stift in eine Ansenkung eingreift. Dadurch kann der Chirurg erkennen, ob eine vorgegebene Verbindung zwischen dem Zielgerät und dem Nagel vorliegt. Eine Fehlbedienung wird verhindert.

Die Erfindung sieht vor, dass die Handhabe drehbar am radialen äußeren Ansatz des Halteabschnitts gelagert ist. Vorzugsweise wird diese Handhabe von einem Drehknopf oder dergleichen gebildet. Die Handhabe betätigt einen radialen Abschnitt, der mit einer Nockenfläche zusammenwirkt derart, dass bei einer Drehung der Handhabe aus einer Ausgangsposition, in der das Verriegelungselement in seiner Entriegelungsstellung ist, in einer Drehrichtung das Verriegelungselement in die Aufnahmebohrung hineinbewegt wird. Die an die Entriegelungsposition sich anschließende Nockenfläche weist einen ersten Nockenflächenabschnitt auf, an den sich ein weiterer Nockenflächenabschnitt anschließt. Der Eingriff zwischen dem radialen Abschnitt und der zweiten Nockenfläche erfolgt unter Selbsthemmung. Auf die Handhabe oder den radialen Abschnitt wirkt eine Feder in Richtung Entriegelungsposition. Befindet sich daher der radiale Abschnitt im ersten Nockenflächenabschnitt, wird er, wenn die Handhabe losgelassen wird, diese automatisch in die Entriegelungsposition zurückverstellen und damit auch das Verriegelungselement in die Freigabestellung bringen. Befindet sich hingegen der radiale Abschnitt im zweiten Nockenflächenabschnitt, liegt Selbsthemmung vor, und die eingestellte Verriegelung kann sich nicht von selbst wieder lösen.

Der Erfindung liegt daher die Aufgabe zugrunde, ein Zielgerät für Verriegelungsnägel zu schaffen, mit dem Nägel unterschiedlicher Länge und unterschiedlich angeordneten Querbohrungen eingesetzt werden können.

Diese Aufgabe wird durch die Merkmale des Patentanspruchs 1 gelöst.

Die beschriebene Funktion hat folgenden Vorteil. Befindet sich das Verriegelungselement außerhalb einer Ansenkung des Haltestabes, dann kann die Handhabe zwar um einen gewissen Betrag gedreht werden, es gelingt aber nicht, den radialen Abschnitt in den zweiten Nockenflächenabschnitt hineinzubewegen. Dadurch kommt es automatisch zu einem Rückspringen in die Entriegelungsposition. Dies ist für den Chirurgen sofort erkennbar. Gelangt jedoch das Verriegelungselement in die Ansenkung, dann kann der radiale Abschnitt in den zweiten Nockenflächenabschnitt hineinbewegt werden und bleibt daher in der Verriegelungsstellung.

Es ist von Vorteil, wenn das Verriegelungselement von einer Feder vorgespannt ist, welche das Auffinden einer Ansenkung erleichtert. Eine Ringnut im Bereich der Ansenkungen erleichtert ihr Auffinden ebenfalls.

Nach einer anderen Ausgestaltung der Erfindung ist der radiale Ansatz ringzylindrisch und die Nockenflächenabschnitte werden von mindestens einer Nut im radialen Ansatz gebildet. Die Handhabe greift mit einem radialen Abschnitt in die Nut ein. Der radiale Abschnitt kann nach einer weiteren Ausgestaltung der Erfindung von einem Querstift gebildet sein, der sich durch den Ansatz hindurcherstreckt und vorzugsweise an beiden Enden in der Handhabe befestigt ist. Hierbei sind zwei gleiche Nuten im Ansatz erforderlich. Der Querstift kann sich gleichzeitig durch eine Querbohrung des Verriegelungselements erstrecken, um diesen in Achsrichtung zu verstellen.

In einer anderen Ausgestaltung der Erfindung ist vorgesehen, dass die Aufnahmebohrung in einem ringzylindrischen Bauteil ausgebildet ist, das mittels eines äußeren radialen Zapfens am Zielarm festlegbar ist. Der Zielarm kann z.B. einteilig aus einem geeigneten Kunststoffmaterial oder Metall geformt sein, während das zylindrische Bauteil mit dem Zapfen aus einem anderen Metall besteht.

Das zylindrische Bauteil weist nach einer weiteren Ausgestaltung der Erfindung mindestens ein Fenster auf, durch welches der Haltestab sichtbar ist. Am Haltestab können Zahlen oder dergleichen angebracht sein, welche im Fenster erscheinen, wenn der Haltestab in einer bestimmten Posittion festgelegt ist.

Die Erfindung soll anhand eines in Zeichnungen dargestellten Ausführungsbeispiels näher erläutert werden.
- Fig. 1: zeigt perspektivisch ein Zielgerät nach der Erfindung.
- Fig. 2: zeigt die Seitenansicht des Zielgeräts nach Fig. 1 ohne Haltestab.
- Fig. 3: zeigt einen Längsschnitt durch das Bauteil nach Fig. 2.
- Fig. 4: zeigt vergrößert den eingekreisten Abschnitt 4 der Darstellung nach Fig. 3.
- Fig. 5: zeigt die Seitenansicht eines Bauteils des Zielgeräts nach Fig. 3 in Richtung Pfeil 5.
- Fig. 6: zeigt die Seitenansicht des Bauteils nach Fig. 5 in Richtung Pfeil 6.
- Fig. 7: zeigt einen Schnitt durch die Darstellung nach Fig. 6 entlang der Linie 7-7.
- Fig. 8: zeigt abgewinkelt eine Nut des Bauteils nach den Figuren 5 bis 7.

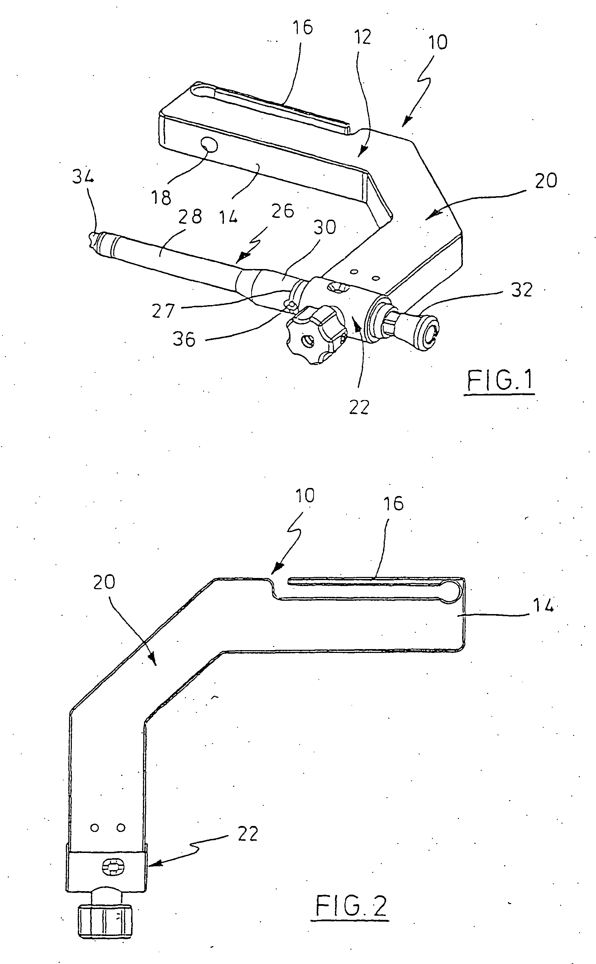

In den Figuren 1 bis 3 ist das Zielgerät allgemein mit 10 bezeichnet. Es weist einen Zielarm 12 auf. Der Zielarm 12 besitzt einen relativ dicken massiven Abschnitt 14 und einen durch einen Schlitz vom massiven Abschnitt 14 getrennten federnden Abschnitt 16. Durch beide hindurch erstreckt sich eine Zielbohrung 18. Mit Hilfe des Federabschnitts 16 kann ein in die Zielbohrung 18 eingeführter Stift oder eine Zielhülse in der angenommenen Lage festgelegt werden. Dieses Prinzip ist jedoch an sich bekannt.

Der Zielarm 12 ist mit einem bügelartigen Abschnitt 20 verbunden, an dessen Ende ein ringzylindrischer Halteabschnitt 22 angebracht ist. Der Halteabschnitt 22 wird anhand der Figuren 5 bis 7 nachfolgend näher erläutert.

Er weist eine Aufnahmebohrung 24 auf, in der ein Haltestab 26 aufgenommen ist. Der Haltestab 26 weist einen ersten zylindrischen Abschnitt 28 auf, an dessen Ende in nicht weiter dargestellter Art und Weise ein Ende eines nicht gezeigten Verriegelungsnagels angebracht werden kann, wie dies etwa in DE-A-202 04 655 dargestellt ist. Der Verriegelungsnagel kann z.B. ein Suprakondylamagel sein. Ein im Durchmesser größerer zylindrischer Abschnitt 30 des Haltestabs 26 erstreckt sich durch die Aufnahmebohrung 24. Am anderen Ende des Haltestabs 26 ist eine Mutter 32 zu erkennen, die auf ein Gewinde eines nicht gezeigten Spannstabes geschraubt ist, der sich durch den hohlen Haltestab 26 hindurcherstreckt. Sein vorderes Ende ist bei 34 angedeutet. Es wird in ein Innengewinde des Verriegelungsnagels eingeschraubt, sodass dieser gegen das linke Ende des Haltestabs 28 gespannt werden kann. Durch geeignete Mittel zwischen Nagel und Haltestab 26 wird der Verriegelungsnagel auch in seiner Drehlage relativ zum Haltestab 26 festgelegt.

Im zylindrischen Abschnitt 30 befinden sich mehrere beabstandete Ansenkungen. Eine ist bei 36 in Fig. 1 angedeutet. Sie liegt auf einer Ringnut 27.

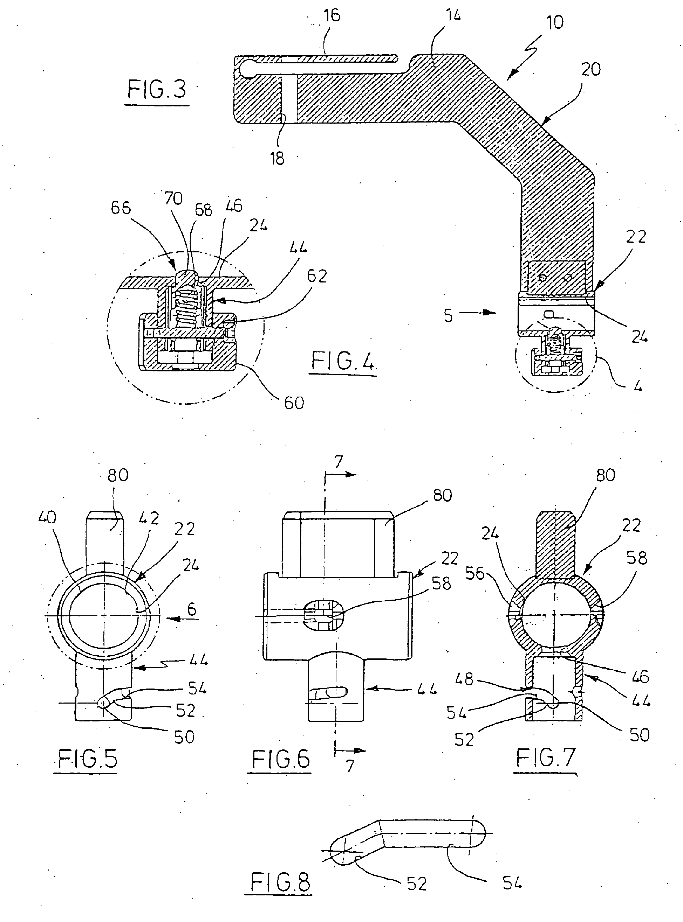

In den Figuren 5 bis 7 ist der zylindrische Halteabschnitt 22 detaillierter dargestellt. Man erkennt, dass die Aufnahmebohrung 24 nicht streng kreiszylindrisch ist, sondern dass an zwei Positionen bei 40, 42 gerade Prismenflächen geformt sind. Diesen Prismenflächen annähernd diametral gegenüberliegend ist außen an dem Halteabschnitt 22 ein hülsenförmiger Ansatz 44 angebracht, der sich über eine Öffnung 46 im zylindrischen Halteabschnitt 22 in die Aufnahmebohrung 24 hineinerstreckt. Wie aus den Figuren 5 bis 7 ferner hervorgeht, befinden sich im Ansatz 44 zwei Nuten 48, die diametral gegenüberliegend angeordnet sind. Die Nuten 48 sind so angeordnet, dass sie mit dem einen Ende einander diametral gegenüberliegen, wie bei 50 in Fig. 5 gezeigt. An diesen Abschnitt schließt sich ein erster Nutabschnitt 52 an, der relativ steil ist. Daran schließt sich ein Nutabschnitt 54 an, der relativ flach ist. In Fig. 8 ist eine derartige Nut in Abwicklung gezeigt. Man erkennt, dass der relativ steile Nutabschnitt 52 einen Steigungswinkel von 35° hat. Der flachere und längere Nutabschnitt 54 weist einen Steigungswinkel von etwa 5° auf.

In den Figuren 6 und 7 ist zu erkennen, dass diametral gegenüberliegend der Halteabschnitt 22 Fenster 56, 58 aufweist, auf deren Funktion später noch eingegangen wird.

In Fig. 4 ist zu erkennen, dass auf dem Ansatz 44 ein Drehknopf 60 drehbar gelagert ist. Der Drehknopf wird diametral von einem Querstift 62 durchsetzt. Der Querstift 62 erstreckt sich durch die beiden Nuten 48. Je nach Lage des Querstiftes 62 in den Nuten 48 befindet sich der Drehknopf 60 in unterschiedlicher axialer Lage auf dem Ansatz 44.

Innerhalb des Ansatzes 44 ist ein Verriegelungselement 66 in Form eines Stiftes axial verschiebbar angeordnet. In seinem in Fig. 4 unteren Bereich ist er hohl ausgeführt. Außerdem weist er eine Querbohrung auf, durch welche sich der Querstift 62 hindurcherstreckt. In einer axialen Bohrung des Verriegelungsstiftes befindet sich eine Schraubenfeder 68. Ein massiver Verriegelungsabschnitt 70 des Verriegelungsstiftes erstreckt sich durch die Öffnung 46 in die Aufnahmebohrung 24 hinein. In Fig. 4 ist die Position des Verriegelungsstiftes gezeigt, in welcher der Abschnitt 70 am weitesten radial in die Aufnahmebohrung 24 hineinsteht. Eine weitere axiale Bewegung wird durch eine nicht bezeichnete äußere Schulter des Verriegelungsstiftes begrenzt, die am Rand der Öffnung 46 anliegt.

In der Entriegelungsposition der beschriebenen Anordnung befindet sich der Querstift 62 im Endbereich der Nuten 48, der mit 50 bezeichnet ist. Der Verriegelungsstift 66 hat daher eine gegenüber Fig. 4 abgesenkte Position, sein Abschnitt 70 ragt jedoch etwas in die Aufnahmebohrung 24 vor. Wird nun der Aufnahmestab 26 nach Fig. 1 in die Aufnahmebohrung 24 eingeführt, kann der Verriegelungsabschnitt 70 in eine Ansenkung 36 einrasten. Wird der Drehknopf 60 dann gedreht, kann der Verriegelungsabschnitt 70 vollständig in die Ansenkung 36 eingreifen. Hierfür ist erforderlich, dass der Drehknopf so weit gedreht wird, dass der Querstift 62 in den Nutabschnitt 54 einläuft. Hat er diesen Abschnitt 54 erreicht, findet eine Selbsthemmung statt, da der Winkel des Nutabschnitts 54 sehr klein ist. Ein automatisches Rückdrehen des Knopfes 60 ist ausgeschlossen.

Wird jedoch der Verriegelungsstift 66 betätigt, ohne dass das Verriegelungselement 68 bereits teilweise in einer Ansenkung 36 eingeschnappt hat, würde eine Verdrehung des Drehknopfes 60 und damit eine Verstellung des Verriegelungsstiftes zu einer Anlage des Verriegelungsabschnitts 70 an der Außenseite des zylindrischen Abschnitts 30 führen. Der Querstift 62 kann hierbei lediglich innerhalb des Nutabschnitts 52 bewegt werden. Den Abschnitt 54 kann er nicht erreichen. Mithin würde der Drehknopf 60 aufgrund der Wirkung der Feder 68 automatisch in die Ausgangsposition zurückgedreht werden, wenn das Verriegelungselement 66 nicht in eine Ansenkung 36 eingreift. Dies kann vom Chirurgen festgestellt werden, sodass eine Fehlbedienung ausgeschlossen ist.

Bei dem beschriebenen Festlegen des Haltestabs 26 in der Aufnahmebohrung 24 wird aufgrund der Verstellung des Verriegelungselements 66 ein radialer Druck auf den Abschnitt 30 des Haltestabs 26 ausgeübt. Dieser wird dadurch gegen die Prismenflächen 40, 42 angepresst, sodass eine sichere Festlegung des Haltestabs 26 erfolgt.

Der Chirurg kann über Fenster 56, 58 in die Aufnahmebohrung 24 hineinsehen und erkennen, ob im Fenster 56, 58 eine Markierung oder eine Zahl erscheint, aus welcher er entnehmen kann, mit welcher Ansenkung 36 das Verriegelungselement 66 in Eingriff gebracht wurde. Da jede Ansenkung 36 einer bestimmten Querbohrung des nicht gezeigten Verriegelungsnagels entspricht, weiß der Chirurg auch, mit welcher Verriegelungsbohrung die Zielbohrung 18 des Zielgeräts 10 ausgerichtet ist.

Der Vollständigkeit halber sei noch erwähnt, dass dem Ansatz 44 gegenüberliegend ein Zapfen 80 am zylindrischen Halteabschnitt 22 angebracht ist, der in einer Ausnehmung des Abschnitts 20 des Zielgeräts 10 eingesetzt und dort sicher befestigt ist.

## Patentansprüche

1. Zielgerät für einen Verriegelungsnagel, der proximal mit Querbohrungen versehen ist, deren Achsen um vorgegebene Abstände und ggf. um vorgegebene Winkel zueinander versetzt angeordnet sind, mit einem mindesten eine Zielbohrung aufweisenden Zielarm (12) und einem am Zielarm (12) angebrachten Halteabschnitt (22) zur Halterung des distalen Endes des Nagels, wobei der Halteabschnitt eine Aufnahmebohrung (24) aufweist, in der ein sich parallel zum Zielarm (14) erstreckender Haltestab (26) geführt ist, der Haltestab (26) Befestigungsmittel aufweist zur Anbringung des Nagels an seinem zugekehrten Ende, der Haltestab (26) im Bereich der Aufnahmebohrung (24) mehrere Ansenkungen (36) aufweist, der Aufnahmebohrung (24) ein radial bewegbares Verriegelungselement (66) zugeordnet ist, das mittels einer Handhabe (60) mit einer der Ansenkungen (36) in Eingriff bringbar ist, um die axiale und Drehlage des Haltestabs (26) in der Aufnahmebohrung (24) festzulegen, wobei die Anordnung der Ansenkungen (36) derart ist, dass die Zielbohrung (18) mit einer Querbohrung des Nagels ausgerichtet ist, wenn das Verriegelungselement (66) in eine Ansenkung (36) eingreift und wobei die Handhabe (60) an einem radialen, hülsenformigen äußeren Ansatz (44) des Halteabschnitts (22) gelagert ist,
**dadurch gekennzeichnet, dass** die Handhabe (60) drehbar gelagert ist und einen radialen Abschnitt betätigt, der mit einer Nockenfläche (48) des Ansatzes (44) zusammenwirkt, derart, dass bei einer Drehung der Handhabe (60) aus einer Ausgangsposition heraus, in der das Verriegelungselement (66) in seiner Entriegelungsstellung ist, in einer vorgegebenen Drehrichtung das Verriegelungselement (66) in die Aufnahmebohrung (24) hineinbewegt wird und die Nockenfläche (48) einen sich an die Entriegelungsposition anschließenden ersten Nockenflächenabschnitt (52) und einen sich an den ersten Nockenflächenabschnitt (52) anschließenden zweiten Nockenflächenabschnitt (54) aufweist, wobei der Eingriff des radialen Abschnitts im zweiten Nockenflächenabschnitt (54) unter Selbsthemmung erfolgt und die Handhabe (60) oder der radiale Abschnitt von einer Feder in Richtung Entriegelungsposition vorgespannt ist.

2. Zielgerät nach Anspruch 1, **dadurch gekennzeichnet, dass** der radiale Ansatz ringzylindrisch ist und die Nockenflächen (52, 54) von einer Nut (48) im radialen Ansatz gebildet sind und der mit der Handhabe (60) verbundene radiale Abschnitt in die Nut (48) eingreift.

3. Zielgerät nach Anspruch 2, **dadurch gekennzeichnet, dass** der radiale Abschnitt von einem Querstift (62) gebildet ist, der sich innerhalb der Nut (48) radial durch den Ansatz erstreckt.

4. Zielgerät nach Anspruch 1 und 3, **dadurch gekennzeichnet, dass** der Verriegelungsstift (66) eine axiale Bohrung aufweist, in der eine Schraubenfeder (68) angeordnet ist, deren anderes Ende sich auf dem Querstift (62) abstützt.

5. Zielgerät nach Anspruch 3 oder 4, **dadurch gekennzeichnet, dass** der Querstift (62) sich durch eine Querbohrung des Verriegelungsstifts (66) erstreckt.

6. Zielgerät nach einem der Ansprüche 1 bis 5, **dadurch gekennzeichnet, dass** der Halteabschnitt (22) von einem ringzylindrischen Bauteil gebildet ist, das mittels eines radialen äußeren Zapfens (80) in einer Ausnehmung des Zielarms (14) festlegbar ist.

7. Zielgerät nach Anspruch 6, **dadurch gekennzeichnet, dass** der Halteabschnitt (22) mindestens ein Fenster (56, 58) aufweist, durch welches der Haltestab (26) sichtbar ist.

8. Zielgerät nach einem der Ansprüche 1 bis 7, **dadurch gekennzeichnet, dass** die Aufnahmebohrung(24) Prismenflächen (40, 42) aufweist, die dem Verriegelungselement (66) annähernd gegenüberliegen und gegen welche der Haltestab (26) vom Verriegelungselement (66) angepresst wird.

9. Zielgerät nach einem der Ansprüche 1 bis 8, **dadurch gekennzeichnet, dass** der Haltestab (26) im Bereich der Ansenkungen jeweils eine Ringnut (27) aufweist, mit der das Verriegelungselement (66) in Eingriff bringbar ist.

## Claims

1. Target device for a locking nail provided proximally with cross bores the axes of which are arranged offset to each other by prespecified distances and optionally prespecified angles with a targeting arm (12) comprising at least one targeting bore and a holding portion (22) attached to the targeting arm (12) for holding the distal end of the nail, whereby the holding portion has a reception bore (24) in which a retaining bar (26) extending parallel to the targeting arm (14) is guided, the retaining bar (26) has fastening means for fixing the nail to its facing end, the retaining bar (26) has a plurality of recesses (36) in the region of the reception bore (24), the reception bore (24) is assigned a radially movable locking element (66) which may be brought into engagement with one of the recesses (36) by means of a handle (60) in order to define the axial and rotational position of the retaining bar (26) in the reception bore (24), whereby the arrangement of the recesses (36) is such that the targeting bore (18) is aligned with a cross bore of the nail when the locking element (66) engages in a recess (36) and whereby the handle (60) is mounted on a radial sleeve-shaped outer lug (44) of the holding portion (22), **characterised in that** the handle (60) is mounted rotatably and actuates a radial portion which interacts with a cam surface (48) of the lug (44) in such way that when the handle (60) is rotated from an initial position in which the locking element (66) is in its unlocking position, the locking element (66) is moved into the reception bore (24) by a predetermined direction of rotation and the cam surface (48) has a first cam surface portion (52) adjoining the unlocked position and a second cam surface portion (54) adjoining the first cam surface portion (52), whereby the engagement of the radial portion in the second cam surface portion (54) takes place in a self-locking manner and the handle (60) or the radial portion is pretensioned by a spring in the direction of the unlocked position.

2. Targeting device according to claim 1, **characterised in that** the radial lug is annularly cylindrical and the cam surfaces (52, 54) are formed by a groove (48) in the radial lug and the radial portion connected to the handle (60) engages the groove (48).

3. Targeting device according to claim 2, **characterised in that** the radial portion is formed by a cross pin (62) which extends radially through the lug within the groove (48).

4. Targeting device according to claim 1 and 3 **characterised in that** the locking pin (66) has an axial bore in which a helical spring (68) is arranged, the other end of which is supported on the cross pin (62).

5. Targeting device according to claim 3 or 4, **characterised in that** the cross pin (62) extends through a cross bore of the locking pin (66).

6. Targeting device according to any one of claims 1 to 5 **characterised in that** the holding portion (22) is formed by an annularly cylindrical component which may be located by means of a radial outer peg (80) in a recess of the target arm (14).

7. Targeting device according to claim 6, **characterised in that** the holding portion (22) comprises at least one window (56, 58) through which the retaining bar (26) can be seen.

8. Targeting device according to any one of claims 1 to 7, **characterised in that** the reception bore (24) comprises prismatic surfaces (40, 42) which are approximately opposite the locking element (66) and against which the retaining bar (26) is pressed by the locking element (66).

9. Targeting device according to any one of claims 1 to 8, **characterised in that**, in the areas of the recesses, the retaining bar (26) has in each case an annular groove (27) with which the locking element (66) can be engaged.

## Revendications

1. Mire pour une broche de verrouillage, munie sur l'extrémité proximale de trous transversaux dont les axes sont disposés en déport mutuel de distances prédéfinies et éventuellement d'angles prédéfinis, comprenant un bras de visée (12) qui présente au moins un trou de visée et une section de retenue (22), montée sur le bras de visée (12), pour la fixation de l'extrémité distale de la broche, la section de retenue présentant un trou de réception (24) dans lequel est guidée une tige de retenue (26) s'étendant parallèlement au bras de visée (14), la tige de retenue (26) présentant des moyens de fixation pour monter la broche sur son extrémité tournée vers cette dernière, la tige de retenue (26) présentant dans la zone du trou de réception (24) plusieurs fraisages (36), un élément de verrouillage (66) mobile dans la direction radiale étant associé au trou de réception (24), lequel élément peut être amené en prise au moyen d'une manette (60) avec l'un des fraisages (36), pour fixer la position axiale et de rotation de la tige de retenue (26) dans le trou de réception (24), l'agencement des fraisages (36) étant tel que le trou de visée (18) s'aligne sur un trou transversal de la broche lorsque l'élément de verrouillage (66) s'engage dans un fraisage (36), et la manette (60) étant montée sur un embout extérieur radial (44), en forme de manchon, de la section de retenue (22), **caractérisée en ce que** la manette (60) est montée tournante et actionne une section radiale, qui coopère avec une surface de came (48) de l'embout (44) de telle sorte que, lors d'une rotation de la manette (60) dans une position de rotation prédéfinie, à partir d'une position initiale dans laquelle l'élément de verrouillage (66) se situe dans sa position de déverrouillage, l'élément de verrouillage (66) est déplacé dans le trou de réception (24) et la surface de came (48) présente une première section de surface (52) se raccordant à la position de verrouillage et une seconde surface de section (54) se raccordant à la première section de surface (52), la prise de la section radiale dans la seconde section de surface de came (54) s'effectuant par auto-blocage et la manette (60) ou par précontrainte de la section radiale par un ressort en direction de la position de déverrouillage.

2. Mire selon la revendication 1, **caractérisée en ce que** l'embout radial est cylindrique circulaire et les surfaces de came (52, 54) sont formées par une gorge (48) dans l'embout radial et la section radiale assemblée s'engage dans la gorge (48) avec la manette (60).

3. Mire selon la revendication 2, **caractérisée en ce que** la section radiale est formée par une goupille transversale (62), qui s'étend radialement au travers de l'embout à l'intérieur de la gorge (48).

4. Mire selon les revendications 1 et 3, **caractérisée en ce que** la goupille de verrouillage (66) comporte un trou axial, dans lequel est disposé un ressort hélicoïdal (68), dont l'autre extrémité s'appuie sur la goupille transversale (62).

5. Mire selon l'une des revendications 3 ou 4, **caractérisée en ce que** la goupille transversale (62) s'étend au travers d'un trou transversal de la goupille de verrouillage (66).

6. Mire selon l'une quelconque des revendications 1 à 5, **caractérisée en ce que** la section de retenue (22) est formée par un composant cylindrique circulaire, qui est fixé dans un creux du bras de visée (14) au moyen d'un tourillon radial extérieur (80).

7. Mire selon la revendication 6, **caractérisée en ce que** la section de retenue (22) comporte au moins une fenêtre (56, 58), au travers de laquelle est visible la tige de retenue (26).

8. Mire selon l'une quelconque des revendications 1 à 7, **caractérisée en ce que** le trou de réception (24) présente des surfaces prismatiques (40, 42), qui se situent à peu près en vis-à-vis de l'élément de verrouillage (66) et contre lesquelles est pressée la tige de retenue (26) par l'élément de verrouillage (66).

9. Mire selon l'une quelconque des revendications 1 à 8, **caractérisée en ce que** la tige de retenue (26) comporte spécifiquement, dans la zone de fraisage, une gorge annulaire (27), avec laquelle l'élément de verrouillage (66) est amené en prise.
